# FASCICULE DE BREVET EUROPEEN

(11) **EP 1 280 890 B1**
(45) Date de publication et mention de la délivrance du brevet: **03.01.2007**
(21) Numéro de dépôt: 01929739.9
(22) Date de dépôt: 27.04.2001
(51) Int. Cl.: C12N 9/00, C12N 1/20, A61K 38/16, C12P 21/02, C12N 5/10

(54) **SOUCHES MUTANTES CAPABLES DE PRODUIRE DES PROTEINES CHIMIQUEMENT DIVERSIFIEES PAR INCORPORATION D'ACIDES AMINES NON CONVENTIONNELS**
MUTANTENSTÄMME GEEIGNET ZUR HERSTELLUNG VON CHEMISCH UNTERSCHIEDLICHEN PROTEINEN, DIE NICHT-KONVENTIONELLE AMINOSÄUREN ENTHALTEN
MUTANT STRAINS CAPABLE OF PRODUCING CHEMICALLY DIVERSIFIED PROTEINS BY INCORPORATION OF NON-CONVENTIONAL AMINO ACIDS

(30) Priorité: 28.04.2000 FR 0005547
(43) Date de publication de la demande: 05.02.2003
(73) Titulaire: INSTITUT PASTEUR, 75015 Paris (FR)
(72) Inventeur: MARLIERE, Philippe, F-91450 Etiolles (FR); DORING, Volker, F-75015 Paris (FR); MOOTZ, Henning, 42781 Haan (DE)
(74) Mandataire: Santarelli
(86) Numéro de dépôt international: PCT/FR2001/001306
(87) Numéro de publication internationale: WO 2001/083718

(56) Documents cités:
- WO-A-00/24922
- IBBA ET AL.: "Towards engineering proteins by site-directed incorporation in vivo of non-natural amino acids" BIOTECHNOLOGY, vol. 12, no. 7, juillet 1994 (1994-07), pages 678-682, XP002158041
- LEMEIGNAN B ET AL: "Phenotypic suppression by incorporation of an alien amino acid" JOURNAL OF MOLECULAR BIOLOGY, LONDON, GB, vol. 231, no. 2, mai 1993 (1993-05), pages 161-166, XP002113371 ISSN: 0022-2836
- BRUNNER: "Biosynthetic incorporation of non-natural amino acids into proteins" CHEMICAL SOCIETY REVIEWS, vol. 22, no. 3, juin 1993 (1993-06), pages 183-189, XP000978937
- TOTH ET AL.: "A mutation in the small (alpha) subunit of glycyl-tRNA synthetase affects amino acid activation and subunit association parameters" THE JOURNAL OF BIOLOGICAL CHEMISTRY, vol. 265, no. 2, 15 janvier 1990 (1990-01-15), pages 1005-1009, XP002158042
- LIN LAURA ET AL: "Mutational analysis suggests the same design for editing activities of two tRNA synthetases." BIOCHEMISTRY, vol. 35, no. 17, 1996, pages 5596-5601, XP002158043 ISSN: 0006-2960
- DORING VOLKER ET AL: "Reassigning cysteine in the genetic code of Escherichia coli." GENETICS, vol. 150, no. 2, octobre 1998 (1998-10), pages 543-551, XP002158044 ISSN: 0016-6731

### LISTE DE SEQUENCES

<110> INSTITUT PASTEUR
<120> SOUCHES MUTANTES CAPABLES DE PRODUIRE DES PROTEINES CHIMIQUEMENT DIVERSIFIEES PAR INCORPORATION D'ACIDES AMINES NON CONVENTIONNELS
<130> D18870
<150> FR 00 05547
   <151> 2000-04-28
<160> 8
<170> PatentIn Ver. 2.1
<210> 1
   <211> 49
   <212> ADN
   <213> Séquence artificielle
<220>
   <223> Description de la séquence artificielle: Oligonucléotide phosphorylé en position 5' dérivé de la séquence du gène codant pour la thymidylate synthase
<400> 1
   tggataaaat ggcgctggca ccggtacatg cattcttcca gttctatgt 49
<210> 2
   <211> 49
   <212> ADN
   <213> Séquence artificielle
<220>
   <223> Description de la séquence artificielle:
   Oligonucléotide phosphorylé en position 5' dérivé de la séquence du gène codant pour la thymidylate synthase
<400> 2
   tggataaaat ggcgctggca ccgatacatg cattcttcca gttctatgt 49
<210> 3
   <211> 18
   <212> ADN
   <213> Séquence artificielle
<220>
   <223> Description de la séquence artificielle:
   Oligonucléotide dérivé de la séquence du gène codant pour la thymidylate synthase
<400> 3
   ggtgtgatca tgatggtc 18
<210> 4
   <211> 18
   <212> ADN
   <213> Séquence artificielle
<220>
   <223> Description de la séquence artificielle: Oligonucléotide dérivé de la séquence du gène codant pour la thymidylate synthase
<400> 4
   cctgcaagat ggattccc 18
<210> 5
   <211> 19
   <212> ADN
   <213> Séquence artificielle
<220>
   <223> Description de la séquence artificielle:
   Oligonucléotide dérivé de la séquence du gène codant pour la thymidylate synthase
<400> 5
   cgcgccgcat tattgtttc 19
<210> 6
   <211> 19
   <212> ADN
   <213> Séquence artificielle
<220>
   <223> Description de la séquence artificielle:
   Oligonucléotide dérivé de la séquence du gène codant pour la thymidylate synthase
<400> 6
   gtctggaccg gtggcgaca 19
<210> 7
   <211> 33
   <212> ADN
   <213> Séquence artificielle
<220>
   <223> Description de la séquence artificielle:
   Oligonucléotide phosphorylé en position 5' dérivé de la séquence du gène codant pour la valyl-tRNA synthétase
<400> 7
   ggggaattcg gtgtgtgaaa ttgccgcaga acg 33
<210> 8
   <211> 33
   <212> ADN
   <213> Séquence artificielle
<220>
   <223> Description de la séquence artificielle:
   Oligonucléotide phosphorylé en position 5' dérivé de la séquence du gène codant pour la valyl-tRNA synthétase
<400> 8
   ggcaagcttc cagtatttca cggggagtta tgc 33

### SEQUENCE LISTING AS ANNEX

<110> INSTITUT PASTEUR
<120> Souches mutantes capables de produire des protéines chimiquement diversifiées par incorporation d'acides aminés non conventionnels
<130> BIF 023252 PCT
<140> PCT/FR01/01306
   <141> 2001-04-27
<150> FR 00 05547
   <151> 2000-04-28
<160> 8
<170> PatentIn version 3.2
<210> 1
   <211> 49
   <212> DNA
   <213> Artificial
<220>
   <223> Oligonucléotide phosphorylé en position 5' dérivé de la séquence du gène codant pour la thymidilate synthétase.
<400> 1
   tggataaaat ggcgctggca ccggtacatg cattcttcca gttctatgt 49
<210> 2
   <211> 49
   <212> DNA
   <213> Artificial
<220>
   <223> Oligonucléotide phosphorylé en position 5' dérivé de la séquence du gène codant pour la thymidilate synthétase.
<400> 2
   tggataaaat ggcgctggca ccgatacatg cattcttcca gttctatgt 49
<210> 3
   <211> 18
   <212> DNA
   <213> Artificial
<220>
   <223> Oligonucléotide phosphorylé dérivé de la séquence du gène codant pour la thymidilate synthétase.
<400> 3
   ggtgtgatca tgatggtc 18
<210> 4
   <211> 18
   <212> DNA
   <213> Artificial
<220>
   <223> Oligonucléotide phosphorylé dérivé de la séquence du gène codant pour la thymidilate synthétase.
<400> 4
   cctgcaagat ggattccc 18
<210> 5
   <211> 19
   <212> DNA
   <213> Artificial
<220>
   <223> Oligonucléotide phosphorylé dérivé de la séquence du gène codant pour la thymidilate synthétase.
<400> 5
   cgcgccgcat tattgtttc 19
<210> 6
   <211> 19
   <212> DNA
   <213> Artificial
<220>
   <223> Oligonucléotide phosphorylé dérivé de la séquence du gène codant pour la thymidilate synthétase.
<400> 6
   gtctggaccg gtggcgaca 19
<210> 7
   <211> 33
   <212> DNA
   <213> Artificial
<220>
   <223> Oligonucléotide phosphorylé en position 5' dérivé de la séquence du gène codant pour la valyl-tRNA synthétase.
<400> 7
   ggggaattcg gtgtgtgaaa ttgccgcaga acg 33
<210> 8
   <211> 33
   <212> DNA
   <213> Artificial
<220>
   <223> Oligonucléotide phosphorylé en position 5' dérivé de la séquence du gène codant pour la valyl-tRNA synthétase.
<400> 8
   ggcaagcttc cagtatttca cggggagtta tgc 33

### SEQUENCE LISTING

<110> INSTITUT PASTEUR
<120> Souches mutantes capables de produire des protéines chimiquement diversifiées par incorporation d'acides aminés non conventionnels
<130> BIF 023252 PCT
<140> PCT/FR01/01306
   <141> 2001-04-27
<150> FR 00 05547
   <151> 2000-04-28
<160> 8
<170> PatentIn version 3.2
<210> 1
   <211> 49
   <212> DNA
   <213> Artificial
<220>
   <223> Oligonucléotide phosphorylé en position 5' dérivé de la séquence du gène codant pour la thymidilate synthétase.
<400> 1
   tggataaaat ggcgctggca ccggtacatg cattcttcca gttctatgt 49
<210> 2
   <211> 49
   <212> DNA
   <213> Artificial
<220>
   <223> Oligonucléotide phosphorylé en position 5' dérivé de la séquence du gène codant pour la thymidilate synthétase.
<400> 2
   tggataaaat ggcgctggca ccgatacatg cattcttcca gttctatgt 49
<210> 3
   <211> 18
   <212> DNA
   <213> Artificial
<220>
   <223> Oligonucléotide phosphorylé dérivé de la séquence du gène codant pour la thymidilate synthétase.
<400> 3
   ggtgtgatca tgatggtc 18
<210> 4
   <211> 18
   <212> DNA
   <213> Artificial
<220>
   <223> Oligonucléotide phosphorylé dérivé de la séquence du gène codant pour la thymidilate synthétase.
<400> 4
   cctgcaagat ggattccc 18
<210> 5
   <211> 19
   <212> DNA
   <213> Artificial
<220>
   <223> Oligonucléotide phosphorylé dérivé de la séquence du gène codant pour la thymidilate synthétase.
<400> 5
   cgcgccgcat tattgtttc 19
<210> 6
   <211> 19
   <212> DNA
   <213> Artificial
<220>
   <223> Oligonucléotide phosphorylé dérivé de la séquence du gène codant pour la thymidilate synthétase.
<400> 6
   gtctggaccg gtggcgaca 19
<210> 7
   <211> 33
   <212> DNA
   <213> Artificial
<220>
   <223> Oligonucléotide phosphorylé en position 5' dérivé de la séquence du gène codant pour la valyl-tRNA synthétase.
<400> 7
   ggggaattcg gtgtgtgaaa ttgccgcaga acg 33
<210> 8
   <211> 33
   <212> DNA
   <213> Artificial
<220>
   <223> Oligonucléotide phosphorylé en position 5' dérivé de la séquence du gène codant pour la valyl-tRNA synthétase.
<400> 8
   ggcaagcttc cagtatttca cggggagtta tgc 33

## Description

La présente invention a pour objet des cellules procaryotes mutantes, notamment *E.coli,* capables de produire des protéines dont les séquences d'acides aminés comprennent au moins un acide aminé non conventionnel, des procédés de production et de purification desdites protéines ainsi que les protéines obtenues par les procédés selon l'invention. L'invention comprend également les applications desdites cellules et protéines dans différents domaines tels que le domaine thérapeutique, cosmétique, diagnostic ou de la biosynthèse ou la biodégradation de composé organique.

Un nombre croissant de protéines produites massivement par des organismes recombinants sont employées comme catalyseurs dans l'industrie chimique ou comme agents thérapeutiques. La recherche de nouvelles protéines aux fonctions diversifiées est l'objet d'une activité intense, soit en criblant les protéines d'organismes extrêmophiles, soit en créant des variants protéiques par mutagénèse et criblage. Toutefois, la variabilité chimique des protéines qui peuvent être produites dans des organismes vivants reste limitée par l'invariance du code génétique, c'est-à-dire restreinte aux combinaisons d'un jeu canonique de 20 acides aminés. Si la descendance des espèces naturelles pouvait être progressivement remodelée dans le laboratoire de manière à adopter différents codes génétiques, l'évolution des protéines pourrait être redirigée et des sources artificielles de biodiversité ainsi établies.

La déviation expérimentale du code génétique est la seule voie qui permettrait de surmonter cette limitation. Un autre code génétique pourrait spécifier un ensemble plus ou moins grand d'acides aminés, un ensemble substitué par des monomères non canoniques ou un ensemble d'acides aminés canoniques parmi lesquels les codons sont redistribués. La spécification d'acides aminés supplémentaires dans des lignées vivantes se prêterait à de multiples applications dont la plus générique serait l'établissement d'une biodiversité artificielle.

L'incorporation, permanente ou transitoire, d'un seul acide aminé supplémentaire, portant un motif chimique qui pourrait réagir sans modifier les acides aminés conventionnels, suffirait à fonder de nouvelles méthodes de fonctionnalisation de protéine. Ceci est justement l'objet de la présente invention.

Döring Volker et al. "Reassigning Cysteine in the Genetic Code of *Eschericchia coli*", Genetics (Octobre 1998), vol. 150, pp 543-551 décrit des cellules de E.coli obtenues par une méthode permettant à des cellules d'acquérir la capacité de produire une protéine dont la séquence en acides aminés comprend au moins un acide aminé différent (méthionine ou isoleucine) de celui normalement présent selon l'information génétique portée par le gène correspondant à ladite protéine ou un acide aminé non conventionnel (O-méthylthréonine ou norleucine) à la place d'une cystéine.

L'invention a pour objet cellule obtenue par une méthode permettant à des cellules d'acquérir la capacité de produire une protéine dont la séquence d'acides aminés comprend au moins un acide aminé non conventionnel à la place d'une valine normalement présente, comprenant les étapes suivantes :
a) la transformation desdites cellules par au moins une introduction d'une mutation faux-sens au niveau d'un codon cible d'un gène codant pour une protéine nécessaire à la croissance desdites cellules, ladite protéine synthétisée à partir du gène ainsi muté n'étant plus fonctionnelle ;
b) le cas échéant la culture des cellules obtenues à l'étape a) dans un milieu de culture contenant le nutriment exigé par la perte de fonctionnalité de ladite protéine ainsi mutée ; et
c) la culture des cellules obtenues à l'étape a) ou b) dans un milieu de culture contenant l'acide aminé codé par ledit codon cible,
caractérisée en ce qu'elle est choisie parmi les cellules suivantes déposées à la CNCM (Collection Nationale de Culture de Microorganismes, Paris, France)
a) souche *E*. *coli* déposée à la CNCM sous le N° I-2468 le 28 avril 2000,
b) souche *E*. *coli* déposée à la CNCM sous le N° I-2469 le 28 avril 2000, et
c) souche *E*. *coli* déposée à la CNCM sous le N° I-2470 le 28 avril 2000.

On entendra désigner dans la présente description par le terme de protéine également les peptides ou les polypeptides, ainsi que les glycoprotéines correspondantes lorsque lesdites protéines sont glycosylées.

On entendra également désigner dans la présente description par acide aminé non conventionnel tout acide aminé autre que les acides aminés incorporés par les ribosomes au cours de la biosynthèse des protéines synthétisées par les organismes unicellulaires ou pluricellulaires procaryotes ou eucaryotes, ainsi que tout acide aminé incorporé à la place de l'acide aminé devant être normalement incorporé à cette place au regard de la séquence nucléique traduite.

On entendra également désigner dans la présente description par mutation faux-sens, une mutation qui transforme un codon qui représente un acide aminé en un codon qui code pour un autre acide aminé, ce dernier, le cas échéant, ne pouvant remplacer l'acide aminé d'origine pour donner une protéine fonctionnelle dans la protéine à la place du résidu d'acide aminé d'origine.

On entendra également désigner dans la présente description par protéine nécessaire à la croissance de cellules, une protéine qui lorsqu'elle est synthétisée par les cellules de manière fonctionnelle permet auxdites cellules de croître dans des conditions de culture données et qui lorsqu'elle est synthétisée par les cellules de manière non fonctionnelle nécessite l'introduction d'un nutriment supplémentaire dans ledit milieu de culture donné pour permettre auxdites cellules de croître. De telles protéines non fonctionnelles peuvent être par exemple synthétisées par des cellules suite à des mutations conditionnelles telles qu'une mutation de type photosensible.

Afin d'illustrer par un exemple, mais sans s'y limiter, on peut citer notamment la protéine thymidylate synthase de *E. coli* qui présente un site catalytique occupé par la cystéine au niveau de la position 146 de sa séquence d'acides aminés et dont les mutations correspondantes du gène (*thyA*) causent une exigence nutritionnelle pour la thymine ou la thymidine, aucun autre acide aminé ne pouvant remplacer la cystéine à ce site.

On entendra désigner dans la présente description par codon cible, le codon de trois bases nucléotidiques transformé par la mutation faux-sens, ledit codon cible étant la séquence de 3 bases avant transformation par ladite mutation faux-sens.

Selon une mise en oeuvre de l'invention, le milieu de culture de l'étape c) ne contient pas le nutriment exigé par la perte de fonctionnalité de ladite protéine mutée.

Selon l'invention, l'étape c) de culture desdites cellules peut comprendre une série de cultures desdites cellules dans un milieu de culture contenant l'acide aminé codé par ledit codon cible (avant sa transformation par ladite mutation faux-sens), chacune desdites cultures de la série étant effectuée jusqu'à obtention de la phase stationnaire de croissance et suivie d'un lavage des cellules obtenues, le nombre de cultures de la série étant suffisant pour permettre la sélection de mutations augmentant la suppression de ladite mutation faux-sens dudit gène muté et la propagation de l'allèle correspondant audit gène muté.

Selon une autre mise en oeuvre de l'invention, la mutation faux-sens est choisie parmi les mutations faux-sens qui ne réversent spontanément qu'à une très faible fréquence, de l'ordre d'un organisme parmi au moins 10¹⁵.

De préférence, la mutation faux-sens sera choisie parmi les mutations faux-sens qui transforment un codon cible d'un gène codant pour une protéine nécessaire à la croissance de ladite cellule en un codon qui comparativement au codon cible présente un changement d'au moins deux bases, de manière plus préférée trois bases.

On préfère également utiliser les méthodes selon l'invention, caractérisées en ce que le codon cible code pour un acide aminé de faible volume stérique et/ou amphiphile et/ou de volume stérique inférieur ou sensiblement égal au volume stérique de l'acide aminé codé par la mutation faux-sens.

Parmi les codons cibles, on préfère notamment les codons cibles codant pour la cystéine et les mutations faux-sens choisies parmi les mutations faux-sens qui transforment un codon cible en un codon codant pour la valine ou l'isoleucine.

Selon une autre mise en oeuvre de l'invention, l'étape a) de transformation desdites cellules est réalisée au moyen d'un vecteur comprenant une séquence dudit gène codant pour une protéine nécessaire à la croissance desdites cellules comportant ladite mutation faux-sens, notamment au moyen d'un vecteur plasmidique.

De tels vecteurs seront préparés selon les méthodes couramment utilisées par l'homme du métier, et les clones en résultant peuvent être introduits dans lesdites cellules par des méthodes usuelles de recombinaison génétique telles que par exemple la lipofection, l'électroporation ou le choc thermique.

Sous un autre aspect, la méthode de sélection de cellules capables de produire une protéine dont la séquence d'acides aminés comprend au moins un acide aminé non conventionnel est caractérisée en ce qu'elle comprend les étapes a), le cas échéant b), et c) d'une méthode selon l'invention, et la sélection des cellules capables de croître à l'étape c).

De manière préférée, la méthode de sélection de cellules selon l'invention, comprendra en outre une étape d) de culture des cellules obtenues à l'étape c) dans un milieu de culture contenant ledit acide aminé codé par ledit codon cible, la concentration dudit acide aminé pouvant être à une concentration supérieure à la concentration dudit acide aminé utilisée à l'étape c), et le choix des cellules sensibles à la concentration dudit acide aminé utilisée à l'étape d).

On entend désigner par cellule sensible à un composé chimique ou biochimique ou à une concentration donnée dudit composé, une cellule dont la croissance est partiellement ou totalement inhibée lorsqu'elle est cultivée dans un milieu de culture contenant ledit composé chimique ou biochimique ou ladite concentration dudit composé.

L'invention comprend également une méthode de sélection de cellules selon l'invention, caractérisée en ce que l'aminoacyl-tRNA synthétase reconnaissant l'acide aminé codé par ladite mutation faux-sens desdites cellules sélectionnées est capable de charger sur un de ses tRNA associés un acide aminé non conventionnel ou un acide aminé autre que ledit acide aminé codé par ladite mutation faux-sens.

On entendra désigner dans la présente description par tRNA associé, un tRNA qui est reconnu par l'aminoacyl-tRNA synthétase reconnaissant un acide aminé et qui peut transférer ledit acide aminé.

L'invention comprend en outre une méthode de sélection de cellules mutantes selon l'invention, caractérisée en ce que la séquence nucléique du gène codant pour ladite aminoacyl-tRNA synthétase comporte au moins une mutation comparée à la séquence du gène sauvage correspondant, ladite mutation n'ayant pas été introduite par une technique de recombinaison génétique.

L'invention concerne également les cellules procaryotes ou eucaryotes isolées capables de produire une protéine dont la séquence d'acides aminés comprend au moins un acide aminé non conventionnel, caractérisées en ce qu'elles comprennent une aminoacyl-tRNA synthétase reconnaissant un acide aminé donné capable de charger sur un de ses tRNA associés un acide aminé non conventionnel ou un acide aminé autre que ledit acide aminé donné, et en ce que la séquence nucléique du gène codant pour ladite aminoacyl-tRNA synthétase comporte au moins une mutation comparée à la séquence du gène sauvage correspondant, ladite mutation n'ayant pas été introduite par une technique de recombinaison génétique.

Ainsi, l'invention concerne une méthode de sélection de cellules basée sur la constitution par la cellule d'une voie métabolique nécessaire à sa croissance permettant d'obtenir des cellules capables de produire un acyl-tRNA non canonique capable de charger un acide aminé non conventionnel.

La souche *E*. *coli* K12, déposée à la CNCM sous le N° I-2467 et identifiée sous la référence β5419, souche initiale pour effectuer les sélections, est un descendant de la souche MG1655 (wt *E. coli* K12), comportant les caractéristiques suivantes :
- délétion au locus thyA et remplacement par un gène de résistance à l'érythromycine,
- délétion au locus nrdD et remplacement par un gène de résistance à la kanamycine,
- porte un plasmide pTZ18 (col E1 réplicon, bla⁺) avec l'allèle Cys146GUA de la thymidylate synthase.

Les cellules selon l'invention sont choisies parmi les cellules suivantes déposées à la CNCM (Collection Nationale de Culture de Microorganismes, Paris, France) :
a) souche *E*. *coli* déposée à la CNCM sous le N° I-2468 le 28 avril 2000,
b) souche *E. coli* déposée à la CNCM sous le N° 1-2469 le 28 avril 2000, et
c) souche *E*. *coli* déposée à la CNCM sous le N° I-2470 le 28 avril 2000.

La souche *E. coli* K12, déposée à la CNCM sous le N° I-2468 et identifiée sous la référence β5456, est un descendant de la souche MG1655 (wt *E. coli* K12), comportant les caractéristiques suivantes :
- délétion au locus thyA et remplacement par un gène de résistance à l'érythromycine,
- délétion au locus nrdD et remplacement par un gène de résistance à la kanamycine,
- porte un plasmide pTZ18 (col E1 réplicon, bla⁺) avec l'allèle Cys146GUA de la thymidylate synthase,
- porte l'allèle T222P du gène valS, dont l'expression produit une forme mutée de la valyl-tRNA synthase qui charge d'autres acides aminés, naturels et artificiels, sur les tRNA/Val.

La souche *E*. *coli* K12, déposée à la CNCM sous le N° I-2470 et identifiée sous la référence β5520, est un descendant de la souche MG1655 (wt *E*. *coli* K12), comportant les caractéristiques suivantes :
- délétion au locus thyA et remplacement par un gène de résistance à l'érythromycine,
- intégration d'un gène de résistance à la tétracycline au locus cycA30::Tn10,
- porte un plasmide pTZ18 (col E1 replicon, bla⁺) avec l'allèle Cys146GUA de la thymidylate synthase,
- porte l'allèle K277Q du gène valS, dont l'expression produit une forme mutée de la valyl-tRNA synthase qui charge d'autres acides aminés, naturels et artificiels, sur les tRNA/Val.

La souche *E*. *coli* K12, déposée à la CNCM sous le N° I-2469 et identifiée sous la référence β5498, est un descendant de la souche CU505 comportant les caractéristiques suivantes :
- délétion au locus nrdD et remplacement par un gène de résistance à la kanamycine,
- porte l'allèle T222P du gène valS,
- souche de génotype leu-455 galT12 LAM-IN (rmD-rmE)1 DE (ilvE-ilvC) nrdD::kan valS:T222P,
- souche déficiente dans la biosynthèse de la valine et proficiente dans la mésincorporation d'acide L-alpha amino butyrique dans les protéines par substitution de la valine.

L'invention comprend en outre l'utilisation d'une cellule selon l'invention pour la production de protéine, notamment recombinante, dont la séquence d'acides aminés comprend au moins un acide aminé non conventionnel.

Sous un autre aspect, l'invention concerne un procédé de production d'une protéine dont la séquence d'acides aminés comprend au moins un acide aminé non conventionnel, caractérisé en ce qu'il comprend les étapes suivantes :
a) le cas échéant, la sélection d'une cellule par une méthode selon l'invention ;
b) la culture de ladite cellule sélectionnée à l'étape a) ou d'une cellule selon l'invention dans un milieu de culture et des conditions de culture permettant la croissance de ladite cellule ; et
c) l'isolement de ladite protéine comprenant au moins un acide aminé non conventionnel à partir du surnageant de culture et/ou du culot cellulaire obtenu à l'étape b).

Dans un mode de réalisation préféré, la présente invention est relative à un procédé de production d'une protéine dont la séquence d'acides aminés comprend au moins un acide aminé non conventionnel, caractérisé en ce qu'il comprend les étapes suivantes :
a) la culture d'une cellule choisie parmi les cellules suivantes déposées à la CNCM (Collection Nationale de Culture de Microorganismes, Paris, France) :
   - souche *E*. *coli* déposée à la CNCM sous le N° I-2468 le 28 avril 2000 ;
   - souche *E*. *coli* déposée à la CNCM sous le N° I-2469 le 28 avril 2000 ; et
   - souche *E. coli* déposée à la CNCM sous le N° I-2470 le 28 avril 2000 ;
      dans un milieu de culture et des conditions de culture permettant la croissance de ladite cellule ; et
b) l'isolement de ladite protéine comprenant au moins un acide aminé non conventionnel à partir du surnageant de culture et/ou du culot cellulaire obtenu à l'étape b).

Parmi les protéines pouvant être produites par un procédé selon l'invention, on peut mentionner, mais sans s'y limiter, les protéines qui par l'incorporation d'au moins un acide aminé non conventionnel permettent d'obtenir une activité recherchée qu'une protéine dont la séquence comporte uniquement des acides aminés conventionnels ne permet pas d'obtenir. Par activité, on entend désigner de manière générale toute activité telle qu'une activité physiologique ou biologique relative aux organismes uni- ou pluricellulaires, même partielle, comme par exemple une activité structurelle ou biochimique, par exemple enzymatique, antigénique, de type anticorps ou de modulation, de régulation ou d'inhibition d'activité biologique, ou encore telle qu'elle permette sa mise en oeuvre dans un procédé de biosynthèse ou de biodégradation de composés chimiques ou biochimiques.

On peut également mentionner parmi les protéines pouvant être produites par un procédé selon l'invention, les protéines dont l'incorporation d'au moins un acide aminé non conventionnel est effectuée de telle manière qu'il n'en résulte pas de modification approfondie de l'activité biologique de la protéine non modifiée correspondante. Outre l'activité biologique conservée de la protéine non modifiée correspondante, ces protéines selon l'invention présenteront un acide aminé non conventionnel dont les propriétés spécifiques pourront être avantageusement exploitées.

Parmi les propriétés spécifiques conférées par la présence d'un acide aminé non conventionnel, on peut citer en particulier les propriétés liées à la présence d'un groupe fonctionnel sur ledit acide aminé non conventionnel capable de réagir facilement et de manière spécifique avec un composé chimique ou biochimique dans des conditions permettant de ne pas altérer l'activité de la protéine ou évitant la modification des acides aminés conventionnels.

La présence de ce groupe fonctionnel spécifique pourra avantageusement être utilisée par exemple pour :
(i) purifier toute protéine, notamment recombinante, incorporant ledit acide aminé non conventionnel ;
(ii) coupler une telle protéine à un support solide ;
(iii) coupler à une telle protéine des molécules capables d'être détectées, telles que des sondes spectroscopiques de nature variée ;
(iv) coupler à une telle protéine des polymères lipophiles ou hydrophiles permettant de les solubiliser dans des solvants ou de faire écran à la reconnaissance par des anticorps ;
(v) coupler une telle protéine à un polynucléotide ;
(vi) coupler une telle protéine à un composé chimique ou biochimique dont la présence permet d'augmenter, de diminuer, de moduler, de réguler ou de cibler l'activité biologique de ladite protéine, ou de modifier sa biodisponibilité en tant que composé à usage thérapeutique ; ou encore
(vii) fixer de manière permanente à une telle protéine un coenzyme qui sinon diffuserait en solution.

Selon la présente invention, l'incorporation d'au moins un acide aminé non conventionnel pourra porter sur des acides aminés à l'origine d'une spécificité ou de l'activité, ou à l'origine de la conformation structurale, de la charge, de l'hydrophobicité ou de la capacité de multimérisation de la protéine non modifiée correspondante. Ainsi, pourront être créées des protéines d'activité équivalente, augmentée ou diminuée, ou de spécificité équivalente, plus étroite, ou plus large que la protéine à acides aminés conventionnels non modifiée correspondante.

Par protéine non modifiée, on entend désigner la protéine sauvage ou recombinante constituée d'acides aminés conventionnels et dont est issue la protéine comprenant l'acide aminé non conventionnel.

De préférence, le procédé de production selon l'invention est caractérisé en ce que ledit milieu de culture de l'étape b) permettant la croissance de ladite cellule contient ledit acide aminé non conventionnel ou un de ses précurseurs.

Selon un mode particulier, un procédé de production selon l'invention est caractérisé en ce que ledit acide aminé non conventionnel est synthétisé par ladite cellule, la synthèse dudit acide aminé non conventionnel pouvant être augmentée par modification génétique de ladite cellule.

L'invention concerne en outre un procédé de production d'une protéine dont la séquence d'acides aminés comprend au moins un acide aminé non conventionnel selon l'invention, caractérisé en ce que ladite cellule est auxotrophe pour l'acide aminé codé par ledit codon cible.

Sont également compris dans la présente invention, les procédés selon l'invention, caractérisés en ce que ladite cellule comprend un gène d'intérêt homologue ou hétérologue dont la séquence codante comporte au moins un codon cible.

D'une manière générale, le gène d'intérêt codera pour un ARN messager qui sera ensuite traduit en protéine d'intérêt.

Le gène d'intérêt peut être isolé par toute technique conventionnelle telle que clonage, PCR (Polymerase Chain Reaction) ou encore synthétisé chimiquement. Il peut être de type génomique (muni d'un ou plusieurs introns) ou ADN complémentaire (ADNc). La protéine d'intérêt peut être constituée par une protéine mature, un précurseur et, notamment un précurseur destiné à être sécrété et comprenant un peptide signal, une protéine tronquée, une protéine chimère provenant de la fusion de séquences d'origines diverses ou encore une protéine mutée présentant des propriétés biologiques améliorées et/ou modifiées.

De manière générale, le gène d'intérêt homologue ou hétérologue pourra être choisi parmi les gènes codant pour toute protéine utilisable comme composé thérapeutique ou cosmétique, ou comme réactif de diagnostic, ou encore comme composé pouvant être mis en oeuvre dans un procédé de biosynthèse ou de biodégradation.

A titre d'exemples, on peut citer les gènes d'intérêt codant pour les protéines d'intérêt suivantes :
- cytokines ou lymphokines (interférons α, β et γ, interleukines et notamment l'IL-2, l'IL-6, l'IL-10 ou l'IL-12, facteurs nécrosant des tumeurs (TNF), facteurs stimulateurs de colonies (GM-CSF, C-CSF, M-CSF, ...) ;
- récepteurs cellulaires ou nucléaires, notamment ceux reconnus par des organismes pathogènes (virus, bactéries, ou parasites) ou leurs ligands ;
- protéines impliquées dans une maladie génétique (facteur VII, facteur VIII, facteur IX, dystrophine ou minidystrophine, insuline, protéine CFTR (Cystic Fibrosis Transmembrane Conductance Regulator), hormones de croissance (hGH) ;
- enzymes (uréase, rénine, thrombine, ...) ou toutes enzymes impliquées dans le métabolisme ou la biosynthèse des protéines, des lipides, des acides nucléiques, des sucres, des acides aminés, des acides gras ou des nucléotides;
- inhibiteurs d'enzymes (α1-antitrypsine, antithrombine III, inhibiteurs de protéases virales, ...) ;
- composés à effet anti-tumoral capables d'inhiber au moins partiellement l'initiation
ou la progression de tumeurs ou cancers (anticorps, inhibiteurs agissant au niveau de la division cellulaire ou des signaux de transduction, produits d'expression des gènes suppresseurs de tumeurs, par exemple p53 ou Rb, protéines stimulant le système immunitaire, ...) ;
- protéines du complexe majeur d'histocompatibilité des classes I ou II ou protéines régulatrices agissant sur l'expression des gènes correspondants ;
- protéines capables d'inhiber une infection virale, bactérienne ou parasitaire ou son développement (protéines antigéniques ayant des propriétés immunogènes, épitopes antigéniques, anticorps, ...) ;
- toxines telles que la ricine, toxine cholérique, diphtérique, ... ou immunotoxines ;
- marqueurs (β-galactosidase, peroxydase, ...) ; et
- luciférase, GFP (green fluorescent protein), etc..

L'invention comprend en outre un procédé de production d'une protéine selon l'invention, caractérisé en ce que le milieu de culture de l'étape b) comprend en outre les composés nécessaires à l'induction de la synthèse de la protéine codée par ledit gène d'intérêt. Ces composés sont connus de l'homme du métier et dépendent notamment de la cellule et du gène homologue ou hétérologue sélectionnés.

L'invention concerne également un procédé selon l'invention, caractérisé en ce que l'activité biologique de la protéine codée par ledit gène d'intérêt est au moins partiellement conservée après l'incorporation dudit acide aminé non conventionnel au niveau du codon cible dudit gène d'intérêt.

L'invention concerne en outre un procédé selon l'invention, caractérisé en ce que l'acide aminé non conventionnel est choisi parmi les acides aminés non conventionnels de formule I et de configuration L 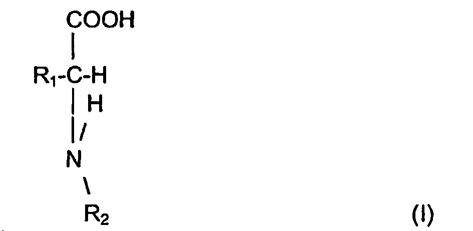 dans laquelle :
R₁ ou R₂ représente des radicaux contenant un groupe fonctionnel capable de réagir de manière sélective, de préférence choisi parmi les groupes aldéhyde, cétone, éthényle, éthynyle ou nitrile.

Parmi ces groupes, on préfère particulièrement le groupe oxo (aldéhyde ou cétone) à réactivité sélective qui faciliterait la fonctionnalisation chimique des protéines. D'autres groupes simples comme le groupe éthynyle se prêteraient également à des réactions sélectives. Un vaste corpus d'expériences conduites à l'aide de systèmes de traduction acellulaire (ex vivo) et d'acyl-tRNAs synthétisés in vitro, a démontré qu'une grande variété de groupes acyles pouvaient être transférés sur le ribosome en réponse à un codon lu par le tRNA. En bref, les modifications latérales des acides aminés semblent toutes compatibles avec la traduction (il n'a à ce jour pas été trouvé d'aminoacide dont la chaîne latérale serait assez encombrante pour bloquer la traduction) ; des substitutions du motif amino en alkyl-amino, en hydroxy et en hydrazino sont compatibles avec la chimie de transpeptidation catalysée par le ribosome (Bain et al. 1991) (il est connu que le ribosome peut former des polyesters en plus des polyamides conventionnels) ; la substitution de l'hydrogène alpha du motif H2NCH(R)-COOH par un groupe alkyle (méthyle) ou l'inversion de configuration au carbone alpha (D-amino-acides) ne sont par contre pas acceptées par le ribosome.

L'invention a également pour objet un procédé selon l'invention, pour la fonctionnalisation de protéine.

L'invention concerne également un procédé de purification de protéine, caractérisé en ce qu'il comprend les étapes suivantes :
a) l'incorporation dans la séquence d'acides aminés de ladite protéine d'un acide aminé non conventionnel contenant un groupe fonctionnel capable de réagir de manière sélective par un procédé selon l'invention ;
b) la mise en contact de la solution contenant la protéine obtenue à l'étape a) avec un support comprenant un composé capable de réagir spécifiquement avec ledit groupe fonctionnel et de fixer spécifiquement ladite protéine ; et
c) l'isolement de ladite protéine fixée sur le support.

Les procédés de purification de protéine, naturelle ou recombinante, utilisés habituellement par l'homme du métier font appel généralement à des méthodes utilisées individuellement ou en combinaison, telles que le fractionnement, les méthodes de chromatographie, les techniques d'immuno-affinité à l'aide d'anticorps mono ou polyclonaux spécifiques, etc.. Ces méthodes sont parfois longues et fastidieuses et ne permettent pas toujours d'obtenir l'activité spécifique, le taux et le rendement de purification voulus. La présence d'un groupe fonctionnel spécifique sur la protéine à purifier capable de réagir sélectivement avec le support de purification sans altérer l'activité de la protéine faciliterait grandement la purification de protéine nécessaire à leur utilisation.

L'invention concerne également un procédé de fixation d'une protéine sur un composé chimique ou biochimique, caractérisé en ce qu'il comprend les étapes suivantes :
a) l'incorporation dans la séquence d'acides aminés de ladite protéine par un procédé selon l'invention d'un acide aminé non conventionnel contenant un groupe fonctionnel capable de réagir de manière sélective ;
b) la mise en contact de la protéine obtenue à l'étape a) avec ledit composé chimique
ou biochimique comprenant un groupe capable de réagir spécifiquement avec ledit groupe fonctionnel dans un milieu permettant la réaction.

De préférence, la fixation d'une protéine sur un composé chimique ou biochimique est une fixation par liaison covalente.

Les composés chimiques ou biochimiques pouvant être utilisés dans ledit procédé de fixation selon l'invention pourront être choisis parmi tous les composés capables de réagir avec le groupe fonctionnel de l'acide aminé non conventionnel incorporé.

On entend désigner dans la présente description par complexe protéique le produit obtenu à l'étape b) du procédé décrit ci-dessus comprenant une protéine selon l'invention fixée sur un composé chimique ou biochimique.

L'invention comprend aussi un procédé selon l'invention, caractérisé en ce que ledit composé chimique ou biochimique est lui-même fixé sur un support solide ou est un composé constitutif d'un support solide.

L'invention concerne en outre un procédé selon l'invention pour la préparation d'un complexe protéique.

De préférence, l'invention comprend les procédés de l'invention, caractérisés en ce que ladite protéine fixée ou ledit composé chimique ou biochimique est choisi parmi les composés thérapeutiques, cosmétiques ou diagnostiques.

Ladite protéine fixée sera choisie en particulier parmi les protéines dont la séquence d'acides aminés comprend un acide aminé non conventionnel selon un procédé de l'invention, et dont la protéine non modifiée correspondante sauvage ou recombinante est choisie parmi les protéines utilisables comme composés thérapeutiques, cosmétiques ou comme réactifs de diagnostic.

De préférence, les procédés selon l'invention sont caractérisés en ce que le composé chimique ou biochimique est choisi parmi les composés capables de modifier l'activité biologique de la protéine fixée.

On entend désigner par composés capables de modifier l'activité biologique d'un autre composé, un composé capable d'augmenter, de diminuer, de réguler l'activité biologique dudit autre composé.

L'invention comprend également un procédé selon l'invention, caractérisé en ce que le composé chimique ou biochimique est choisi parmi les composés dont l'activité biologique peut être modifiée par la protéine fixée.

L'invention comprend également un procédé selon l'invention, caractérisé en ce que le composé chimique ou biochimique est choisi parmi les composés comprenant une protéine, un polynucléotide, un acide gras, un sucre ou un polymère naturel ou synthétique.

Selon un autre aspect, l'invention a pour objet les protéines, en particulier recombinantes, et les complexes protéiques obtenus par un procédé selon l'invention.

Selon la présente invention, les protéines obtenues par un procédé de production de protéine de l'invention seront de nature recombinante et leurs séquences d'acides aminés comprendront au moins un acide aminé non conventionnel.

Selon la présente invention, les complexes protéiques obtenus par un procédé de préparation de complexes protéiques de l'invention seront en particulier caractérisés en ce qu'ils comprennent une protéine recombinante dont la séquence d'acides aminés comprend un acide aminé non conventionnel contenant un groupe fonctionnel, et un composé chimique ou biochimique comprenant un groupe capable de réagir avec ledit groupe fonctionnel.

L'invention comprend également une méthode de sélection de composés capables de se lier à une protéine selon l'invention ou capables de se lier au composé chimique ou biochimique du complexe protéique selon l'invention. Parmi ces méthodes, on peut citer comme exemple une méthode caractérisée en ce qu'elle comprend les étapes suivantes :
a) la mise en contact dudit composé susceptible d'être sélectionné avec la protéine ou le complexe protéique selon l'invention, ladite protéine ou complexe protéique pouvant être notamment fixée sur un support solide ;
b) la détermination de la capacité dudit composé à se lier avec la protéine
ou le complexe protéique selon l'invention.

Les composés susceptibles d'être sélectionnés peuvent être des composés organiques tels que des protéines ou hydrates de carbone ou tous autres composés organiques ou inorganiques déjà connus, ou des composés organiques nouveaux élaborés à partir de techniques de modélisation moléculaire et obtenus par synthèse chimique ou biochimique, ces techniques étant connues de l'homme de l'art.

Les cellules selon l'invention pourront également avantageusement servir de modèle et être utilisés dans des procédés pour étudier, identifier et/ou sélectionner des protéines selon l'invention ou des composés susceptibles de posséder une activité recherchée.

L'invention concerne également l'utilisation d'une protéine ou d'un complexe protéique selon l'invention comme réactif de diagnostic ainsi que les procédés de diagnostic, notamment pour la détection, l'identification, la localisation et/ou le dosage spécifique de polypeptide ou de polynucléotide, mettant en oeuvre une protéine ou un complexe protéique selon l'invention.

Sont en effet comprises dans les protéines selon l'invention, des protéines ayant incorporé au moins un acide aminé non conventionnel et ayant conservé partiellement ou totalement l'activité initiale des protéines sauvages ou recombinantes non modifiées correspondantes, telles que des anticorps, des antigènes, des enzymes ou leurs fragments biologiquement actifs connus pour être utilisés dans des procédés de diagnostic.

De la même manière, sont compris dans les complexes protéiques selon l'invention, des complexes protéiques formés à partir d'une protéine selon l'invention et un composé chimique ou biochimique tels que des complexes comprenant un anticorps, un antigène ou une sonde oligonucléotidique couplé à une enzyme, à un substrat ou à une molécule capable d'être détectée.

Parmi les procédés de diagnostic selon l'invention, on peut citer par exemple les procédés comprenant les étapes suivantes :
a) la mise en contact de l'échantillon biologique susceptible de contenir le composé recherché avec une protéine ou un complexe protéique selon l'invention, ladite protéine ou complexe protéique pouvant être notamment fixée sur un support solide ; et
b) la mise en évidence, l'identification, la localisation et/ou le dosage du complexe formé entre le composé recherché et une protéine ou un complexe protéique selon l'invention.

L'homme du métier saura adapter avec les protéines ou les complexes protéiques selon l'invention les procédés de diagnostic standards connus.

Les techniques et les réactifs spécifiques permettant la mise en évidence, l'identification, la localisation et/ou le dosage du complexe formé que l'on pourra utiliser dans les procédés de l'invention, sont bien connus également de l'homme du métier et sont, par exemple, les techniques ELISA, RIA, d'immunofluorescence, de PCR ou d'autres techniques d'amplification d'un acide nucléique cible connues de l'homme de l'art.

L'invention concerne également un kit ou nécessaire de diagnostic, notamment pour la détection, l'identification, la localisation et/ou le dosage spécifique de protéine ou de polynucléotide caractérisé en ce qu'il contient une protéine ou un complexe protéique selon l'invention.

L'invention concerne en outre l'utilisation d'une protéine, d'un complexe protéique ou d'une cellule selon l'invention pour la préparation d'une composition pharmaceutique ou cosmétique.

L'invention a enfin pour objet une composition pharmaceutique ou cosmétique comprenant une protéine, un complexe protéique ou une cellule selon l'invention.

D'autres caractéristiques et avantages de l'invention apparaissent dans la suite de la description avec les exemples ci-après.

### EXEMPLES

Caractéristiques de souches mentionnées ci-après dans les exemples.

La souche *E*. *coli* K12, déposée à la CNCM sous le N° I-2025 et identifiée sous la référence β5366, est un descendant de la souche MG1655 (wt *E*. *coli* K12), comportant les caractéristiques suivantes :
- délétion au locus thyA et remplacement par un gène de résistance à l'érythromycine,
- porte un plasmide pTZ18 (col E1 replicon, bla⁺) avec l'allèle Cys146GUA de la thymidylate synthase.

La souche *E. coli* K12, déposée à la CNCM sous le N° I-2026 et identifiée sous la référence β8144, est un descendant de la souche MG1655 (wt *E. coli* K12), comportant les caractéristiques suivantes :
- délétion au locus thyA et remplacement par un gène de résistance à l'érythromycine,
- porte un plasmide pTZ18 (col E1 replicon, bla⁺) avec l'allèle Cys146GUA de la thymidylate synthase.

La souche *E. coli* K12, déposée à la CNCM sous le N° I-2027 et identifiée sous la référence β8146, est un descendant de la souche MG1655 (wt *E. coli* K12), comportant les caractéristiques suivantes :
- délétion au locus thyA et remplacement par un gène de résistance à l'érythromycine,
- porte un plasmide pTZ18 (col E1 replicon, bla⁺) avec l'allèle Cys146GUA de la thymidylate synthase.

La souche *E. coli* K12, déposée à la CNCM sous le N° I-2339 et identifiée sous la référence β5479, est un descendant de la souche MG1655 (wt *E. coli* K12), comportant les caractéristiques suivantes :
- délétion au locus thyA et remplacement par un gène de résistance à l'érythromycine,
- délétion au locus nrdD et remplacement par un gène de résistance à la kanamycine,
- porte l'allèle R223H du gène valS,
- porte un plasmide pTZ18 (col E1 replicon, bla⁺) avec l'allèle Cys146GUA de la thymidylate synthase.

La souche *E. coli* K12, déposée à la CNCM sous le N° I-2340 et identifiée sous la référence β5485, est un descendant de la souche MG1655 (wt *E. coli* K12), comportant les caractéristiques suivantes :
- délétion au locus thyA et remplacement par un gène de résistance à l'érythromycine,
- délétion au locus nrdD et remplacement par un gène de résistance à la kanamycine,
- porte l'allèle chromosomique Val 276 Ala du gène valS,
- porte un plasmide pTZ18 (col E1 replicon, bla⁺) avec l'allèle Cys146GUA de la thymidylate synthase.

La souche *E. coli* K12, déposée à la CNCM sous le N° I-2341 et identifiée sous la référence β5486, est un descendant de la souche MG1655 (wt *E. coli* K12) comportant les caractéristiques suivantes :
- délétion au locus thyA et remplacement par un gène de résistance à l'érythromycine,
- délétion au locus nrdD et remplacement par un gène de résistance à la kanamycine,
- porte l'allèle chromosomique Asp 230 Asn du gène valS,
- porte un plasmide pTZ18 (col E1 replicon, bla⁺) avec l'allèle Cys146GUA de la thymidylate synthase.

### EXEMPLE 1 : Construction d'une souche d'E. coli comportant une mutation faux-sens Cys->Val au site actif de la thymidylate synthase et créant une exigence nutritionnelle pour la thymine, la thymidine ou la cystéine.

Les allèles artificiels du gène thyA sont construits par mutagénèse dirigée du plasmide pTS0 (Lemeignan et al., 1993), qui dérive du plasmide pTZ18R (BioRad) par insertion du gène thyA sauvage d'E. coli. La mutagénèse dirigée à l'aide d'un oligonucléotide est réalisée selon la méthode décrite par Kunkel et coll. (1987) sur le phagémide pTS0. La préparation de la matrice simple brin de pTS0, amplifiée dans la souche RZ1032 (Kunkel et coll., 1987) (Hfr KL16 P045 [lysA(61-62)] dut1 ung1 thi1 relA1 supE44 zbd-279::Tn10) est réalisée selon le protocole décrit par Sambrook et coll. (1989). Un oligonucléotide phosphorylé en 5' (acheté à la société Genome Express) est utilisé comme amorce mutagène :
Oligodéoxynucléotide 1 (SEQ ID NO : 1) :
5'pTGGATAAAATGGCGCTGGCACCGGTACATGCATTCTTCCAGTTCTATGT.

L'hybridation de cet oligonucléotide avec la matrice simple brin dans chacune des deux constructions est réalisée avec 10 ng d'oligonucléotide et 0,2 NI de matrice dans un volume de 10 µl d'une solution tampon contenant 20 mM de Tris-HCl pH 7,5, 2 mM d'EDTA et 50 mM de chlorure de sodium. Les tubes sont incubés 5 mn à 70°C puis refroidis progressivement jusqu'à 30°C. A ce mélange est alors ajouté 0,5 mM de chacun des dNTP, 1 mM d'ATP, 10 mM de Tris-HCl à pH 7,5, 10 mM de chlorure de magnésium, 2 mM de dithiothréitol et 1 unité de chacune des deux enzymes du phage T4 ADN ligase et ADN polymérase. Ce mélange réactionnel d'un volume final de 20 µl est incubé 60 min à 37°C dont 5 µl sont ensuite utilisés pour transformer des cellules compétentes de la souche GT869 (Parsot, C. 1986) (thrB1004 pro thi strA hsdS lacZ ΔM15 [F' lacZ ΔM15 laclq traD36 proA+ proB+]) d'E. coli K12 suivant la méthode décrite par Sambrook et coll. (1989). Les cellules transformées sont étalées sur des boîtes de Pétri contenant du milieu LB additionné de 100 mg/l de carbénicilline. Douze clones résistant à l'antibiotique sont réisolés sur le même milieu. L'ADN simple brin correspondant aux phagémides de ces clones est préparé et séquencé selon la méthode didéoxy (Sanger et coll., 1977). Le kit de séquençage M13 (Boehringer Mannheim, Mannheim, Allemagne) et la désoxyadénosine 5'-(α-thio)triphosphate (1300 Ci/mmol, Amersham) sont combinés selon les indications des fournisseurs. Quatre amorces sont utilisées pour déterminer la séquence des allèles de thyA :
Oligodéoxynucléotide 3 (SEQ ID NO : 3) : 5'GGTGTGATCATGATGGTC
Oligodéoxynucléotide 4 (SEQ ID NO : 4) : 5'CCTGCAAGATGGATTCCC
Oligodéoxynucléotide 5 (SEQ ID NO : 5) : 5'CGCGCCGCATTATTGTTTC
Oligodéoxynucléotide 6 (SEQ ID NO : 6) : 5'GTCTGGACCGGTGGCGACA

Le plasmide pTS1 ainsi obtenu propage l'allèle thyA:Val146, dans lequel la position 146 occupée dans le gène thyA sauvage par le codon UGC de la cystéine est occupée par le codon GUA de la valine. Le plasmide pTS1 est introduit par transformation, réalisée selon la méthode de Sambrook et coll. (1989), dans la souche ΔthyA d'*E*. *coli* K12, β1308 (Lemeignan et coll., 1993), dont le gène chromosomique de la thymidylate synthase, thyA, est délété. La souche transformée portant l'allèle plasmidique thyA:Val146, β5366, se montre incapable de croître sans que de la thymine ou de la thymidine soit ajoutée au milieu de culture, tout comme la souche β1308 dont elle dérive. Par contre, la souche β5366 montre une croissance marginale à 30°C sur gradient de diffusion de cystéine, réalisé dans des boîtes de Pétri contenant 25 ml de milieu minéral MS glucose à partir d'un puits central comportant 0,1 ml d'une solution 400 mM de L-cystéine. Dans les mêmes conditions, la souche β1308 ne donne lieu à aucune croissance détectable. Ainsi, la mutation faux-sens convertissant la cystéine catalytique à la position 146 de la thymidylate synthase en valine peut être partiellement supprimée par apport massif de cystéine exogène. L'addition de 0,1 mM de valine au milieu des boîtes de Pétri abolit la croissance de la souche β5366 sur gradient de cystéine. Ainsi, tout se passe comme si la cystéine pouvait s'infiltrer dans le site actif de la valyl-tRNA synthétase pour former des Cys-tRNA^{Val} erronés, aptes à corriger la substitution de la cystéine par la valine dans le site actif de la thymidylate synthase. L'excès de valine préviendrait la formation de ces Cys-tRNA^{Val} erronés.

### EXEMPLE 2 : Construction d'une souche d'E. coli comportant une mutation faux-sens Cys->Ile au site actif de la thymidylate synthase et créant une exigence nutritionnelle pour la thymine, la thymidine ou la cystéine.

La construction correspondante est également conduite pour remplacer la cystéine en position 146 de la thymidylate synthase, par mutagénèse dirigée à l'aide de l'oligonucléotide 2, suivant le même protocole que dans l'Exemple 1.
Oligodéoxynucléotide 2 (SEQ ID NO : 2) :
5'pTGGATAAAATGGCGCTGGCACCGATACATGCATTCTTCCAGTTCTATGT

Le plasmide pTS2 ainsi obtenu propage l'allèle thyA:lle146, dans lequel la position 146 occupée dans le gène thyA sauvage par le codon UGC de la cystéine est occupée par le codon AUA de l'isoleucine. La souche propageant l'allèle plasmidique thyA:lle146, β5274, se révèle exiger l'apport nutritionnel de thymine, de thymidine ou de cystéine en excès, tout comme la souche β5366. La suppression phénotypique de la souche β5274 par la cystéine est abolie par 0,1 mM d'isoleucine, tout comme celle de la souche β5366 par la valine. Ainsi, tout se passe comme si l'isoleucyl-tRNA synthétase était capable de former des Cys-tRNA*^{lle}* erronés en présence d'un excès de cystéine, et que cette formation erronée était prévenue par la présence d'un excès d'isoleucine.

### EXEMPLE 3 : Sélection de mutants du code génétique mésincorporant la cystéine au lieu de la valine par culture sérielle en liquide et caractérisation génétique des mutants de la valyl-tRNA synthétase ainsi obtenus.

La souche β5366 portant l'allèle faux-sens thyA:Val146 sur le plasmide pTS1 est cultivée en milieu minéral MS glucose (2 g/l, Richaud et coll., 1993) supplémenté avec 0,3 mM de thymidine pendant 24 h à 30°C en aérobiose. Les cellules sont ensuite lavées deux fois avec du milieu minéral MS désoxygéné. Un milieu nutritif désoxygéné contenant 10 ml de milieu minéral MS glucose additionné de 1,5 mM de cystéine est inoculé au 1/100 à l'aide des cellules lavées. Les cellules sont ensuite cultivées en anaérobiose pendant 24 h à 30°C et un tube frais contenant 10 ml de milieu minéral MS glucose cystéine désoxygéné est inoculé avec une dilution au 1/100 de la culture en phase stationnaire précédente. Cette procédure est répétée 26 fois. A l'issue de cette propagation sérielle, 12 clones de la culture liquide sont isolés sur boîtes de milieu minéral MS glucose (2 g/l) thymidine (0,3 mM) en aérobiose et sauvegardés en suspension dans le même milieu liquide à - 80°C. Les douze clones sont testés sur des boîtes contenant du milieu minéral MS glucose additionné de facteurs nutritionnels. Tous ces clones se révèlent exiger la thymine ou la thymidine comme facteur de croissance, à moins que de la cystéine soit présente dans le milieu de culture, à une concentration d'au moins 1,5 mM.

De tels clones sont choisis pour une caractérisation génétique approfondie, β8144 et β8146. Des expériences de transduction par le phage P1 du caractère de résistance à la kanamycine, introduit dans le locus nrdD voisin du gène valS de la valyl-tRNA synthétase (97 mn du chromosome d'*E*. *coli* K12) sont effectuées à l'aide des souches β8144 et β8146. Dans les deux cas, environ la moitié des transductants résistant à la kanamycine montrent également une dépendance nutritionnelle pour la thymidine suppressible par la cystéine exogène à la concentration d'au moins 1,5 mM. Cette proportion est en accord avec la distance génétique entre les gènes valS et nrdD (0,4 mn) et laisse supposer que le phénotype de suppression de la mutation faux-sens Cys->Val au site actif de la thymidylate synthase par de faibles concentrations de cystéine est causé par l'altération génétique du locus vals.

La fixation d'une altération génétique dans le gène valS des souches adaptées est confirmée par séquençage de ce locus : un A changé en C cause le remplacement de la lysine à la position 277 par la glutamine dans les deux souches adaptées β8144 et β8146. Le séquençage est effectué sur une matrice obtenue par réaction de polymérisation en chaîne (PCR) réalisée dans des conditions décrites par Sambrook et coll. (1989). La réaction d'amplification est réalisée dans 100 µl d'une solution contenant 10 ng d'ADN génomique des souches β8144 ou β8146, 20 pmoles de chaque amorce, 40 nmoles d'un mélange équimolaire des 4 désoxynucléotides triphosphates, 10 µl d'un tampon composé de 100 mM Tris-HCl pH 8,3, 500 mM KCl et 20 mM MgCl₂, en présence de 1 à 2 unités de Vent polymérase (Biolabs). Pour chaque réaction, 30 cycles de polymérisation sont accomplis, en utilisant un amplificateur d'ADN (Perkin-Elmer Cetus), comme suit : la dénaturation est effectuée à 94°C pendant 5 min pour le 1er cycle et 1 min pour les cycles suivant, l'hybridation à 58°C pendant 1 min et l'élongation à 72°C pendant 3 min pour les 29 premiers cycles et pendant 10 min pour le dernier cycle. Les oligonucléotides 7 et 8 sont utilisés pour l'amplification du gène.
Oligodéoxynucléotide 7 (SEQ ID NO:7) :
5'GGGGAATTCGGTGTGTGAAATTGCCGCAGAACG
Oligodéoxynucléotide 8 (SEQ ID NO : 8) :
5'GGCAAGCTTCCAGTATTTCACGGGGAGTTATGC

Les fragments de PCR ainsi obtenus sont purifiés en utilisant le kit QIAquick (Qiagen) et transmis à la société Genaxis pour en déterminer la séquence.

### EXEMPLE 4 : Suppression phénotypique par des précurseurs métaboliques de la cystéine.

L'exigence nutritionnelle en cystéine des souches adaptées β8144 et β8146 est mise à profit pour caractériser des précurseurs métaboliques qui puissent se substituer à la cystéine dans le milieu de culture sans donner lieu à la dégradation par oxydation. La S-carbamyl-L-cystéine (3 mM), la S-méthyl-L-cystéine (3 mM) et l'acide L-thiazolidine-4-carboxylate (2 mM) se révèlent capables de remplacer la cystéine comme facteur de croissance des souches adaptées β8144 et β8146, au lieu de la thymidine ou de la thymine. Les mêmes composés se révèlent capables de satisfaire l'exigence en cystéine d'un mutant cysN::kan (souche JT1, procurée par M. Berlyn, Coli Genetic Stock Center, Yale University, USA (Levh et al., 1988)). Toutefois, l'addition d'aucune de ces substances ne permet la croissance de la souche β1308 portant une délétion chromosomique du gène thyA de la thymidylate synthase, excluant ainsi leur contamination par des traces de thymine ou de thymidine.

### EXEMPLE 5 : Sélection de mutants du code génétique mésincorporant la cystéine au lieu de la valine par isolement sur milieu solide et caractérisation génétique des mutants de la valyl-tRNA synthétase mésincorporant la cystéine.

La souche β5366 portant l'allèle faux-sens thyA:Val146 sur le plasmide pTS1 est transduite avec un lysat du phage P1 récolté sur une souche auxiliaire d'*E*. *coli* (β7170, Bouzon et al., 1997) dans le chromosome de laquelle un marqueur de résistance à la kanamycine avait été introduit au locus nrdD, voisin du locus valS du gène de la valyl-tRNA synthétase, produisant ainsi la souche β5419. Un allèle mutateur du gène dnaQ est introduit extemporanément par transduction de la souche β5419 à l'aide d'un lysat du phage P1 récolté sur une souche auxiliaire (MS2131, Shapiro, 1990) portant un marqueur de résistance à la tétracycline dnaQ::miniTn10. Un tel clone résistant à la tétracycline et montrant un taux de mutation spontanée amplifié environ 1000 fois (pour l'acquisition de la résistance à la streptomycine) est cultivé à 30°C en milieu minimum glucose en présence de thymidine (0,3 mM). Après 24 h, les cellules sont récoltées, lavées deux fois dans un volume identique de milieu de culture sans thymidine. Un volume de 0,1 ml de la suspension résultante, correspondant à environ 10⁸ bactéries, est étalé à la surface d'une série de boîtes de Pétri contenant une concentration de S-carbamyl-L-cystéine variant entre 0 et 8 mM par incrément de 1 mM additionnant du milieu minéral MS glucose (2 g/l). La même procédure est appliquée à la souche non mutatrice β5419, au gène dnaQ sauvage. L'ensemble des boîtes de Pétri est incubé 96 h à 30°. Des colonies apparaissent sur les boîtes ayant une concentration de S-carbamyl-L-cystéine dépassant 2 mM dans le seul cas où l'allèle mutateur dnaQ::miniTn10 a été introduit dans la souche testée.

Un lysat du phage P1 récolté à partir d'un tel clone est employé pour transduire la souche β5366 portant l'allèle plasmidique thyA:Val146. Environ la moitié des transductants résistant à la kanamycine se montrent capables de croître en présence de 3 mM de S-carbamyl-L-cystéine et en absence de thymine ou de thymidine, parmi lesquels la souche β5455. L'autre moitié des transductants en est incapable et exige la thymine ou la thymidine pour proliférer, tout comme la souche β5366. Cette proportion entre les phénotypes s'accorde avec la distance génétique entre les locus valS et nrdD (0,4 mn). Ainsi, la suppression de la mutation faux-sens de thyA Cys->Val par une faible concentration de cystéine exogène pourrait résulter d'une altération du gène de la valyl-tRNA synthétase. Le locus valS d'une des souches obtenues par transduction de β5366 et capables de croître en présence de 3 mM de S-carbamyl-L-cystéine et en absence de thymine ou de thymidine, désignée β5455, est amplifié par réaction de polymérisation en chaîne et séquencé comme il est décrit dans l'Exemple 3. Un A changé en C cause le remplacement de la thréonine à la position 222 par la proline, confirmant ainsi la fixation d'une altération génétique dans le gène valS de la souche β5455.

### EXEMPLE 6 : Sensibilité des mutants de la valyl-tRNA synthétase à des acides aminés non canoniques.

Les souches β5455, β8144 et β8146 sont testées pour leur sensibilité à des acides aminés artificiels qui présentent une ressemblance stérique avec la valine. Le test est réalisé sur des boîtes de milieu minéral MS glucose supplémenté avec de la thymidine. Les cellules sont cultivées en milieu aérobie (minéral MS glucose 0,3 mM thymidine) pendant 24 h à 30°C et diluées au 1/250 dans du milieu minéral MS. 0,5 ml de cette suspension cellulaire sont étalés sur boîtes de Pétri contenant 25 ml de milieu minéral MS glucose. Un puits est ensuite évidé au centre de la boîte et rempli avec 0,1 ml d'une solution d'un acide aminé :
(1) 100 mM L-2-amino-butyrate
(2) 100 mM L-2-amino-valérate
(3) 100 mM L-2-3-diamino-propionate
(4) 50 mM L-3-thiol-2-amino-butyrate.

Les boîtes sont ensuite incubées pendant 24 h à 30°C et l'apparition éventuelle sur les boîtes d'une zone d'inhibition autour du puits est enregistrée. Les diamètres des zones d'inhibition de croissance atténuées sur des boîtes de Pétri sont mesurés :
L-2-amino-butyrate : 5,2 cm (β5455), 5,7 cm (β8144), 6,7 cm (β8146) ;
L-2-amino-valérate : 2,1 cm (β5455), 1,5 cm (β8144), 6,7 cm (β8146) ;
L-2-3-diamino-propionate : 2,3 cm (β5455), 2,7 cm (β8144), 1,9 cm (β8146)
L-3-thiol-2-amino-butyrate : 2,0 cm (β5366), 4,6 cm (β5455), 4,0 cm (β8144), 4,0 cm (β8146).

Le L-2-amino-butyrate, le L-2-amino-valérate et le L-2,3 diamino-propionate aux concentrations indiquées sont sans effet sur la souche β5366 à l'allèle valS sauvage, mais inhibent la croissance des souches portant un gène valS muté. Le L-3-thiol-2-amino-butyrate inhibe la croissance de toutes les souches, mais on peut remarquer une inhibition plus importante sur les souches mutées. Ainsi, tout se passe comme si les mutants de la valyl-tRNA synthétase avaient une spécificité élargie les rendant capables de charger des tRNA^{Val} avec des acides aminés qui ne peuvent être incorporés par la forme sauvage de l'enzyme.

### EXEMPLE 7 : Incorporation de l'acide aminé non canonique α-aminobutyrate dans les protéines d'une souche d'E. coli mutée dans la valyl-tRNA synthétase.

Un lysat de phage P1 obtenu à partir de la souche β5455 (voir exemple 5), a été employé pour transduire la souche CU505 portant une délétion ÆilvCABD et une mutation leu la rendant auxotrophe pour la valine, l'isoleucine et la leucine. La souche CU505 a été obtenue auprès du Coli Genetic Stock Center, à Yale University (USA). Des clones transductants ont été sélectionnés sur des boîtes LB kanamycine et testés pour leur sensibilité à l'amino-butyrate (3 mM) en milieu solide MS glucose (2 g/l) contenant 0,3 mM de chacun des trois acides aminés valine, isoleucine et leucine. Environ 50 % des clones transductants ne pouvaient croître dans ces conditions, indiquant la co-transduction de l'allèle valS:T222P et le marqueur de résistance nrdD::kan (voir exemple 5). L'un des clones transductants, désigné β5498, a été utilisé pour démontrer l'incorporation d'amino-butyrate en remplacement de la valine, en comparaison avec CU505. Les deux souches ont été cultivées à 30°C en milieu liquide MS glucose (2 g/l) contenant le dipeptide Ile-Leu à la concentration de 0,3 mM et le dipeptide Ile-Val à la concentration de 0,02 mM soit en présence de 0,2 mM de L-amino-butyrate soit en absence de l'analogue. L'inoculum correspondant à chaque souche provenait d'une préculture en milieu liquide MS glucose (2 g/l) contenant le dipeptide Ile-Leu à la concentration de 0,3 mM et le dipeptide Ile-Val à la concentration de 0,04 mM. Les cultures (50 ml) en phase stationnaire après 24 h à 30°C ont été récoltées par centrifugation. Pour chaque test, le culot a ensuite été resuspendu dans 25 ml d'une solution d'acide trichloroacétique à 100 g/l (TCA 10%) à 4°C, centrifugé, resuspendu dans 5 ml de TCA 10 %, centrifugé à nouveau, le culot resuspendu dans du TCA 5 %, la suspension incubée à 95°C pendant 30 mn, centrifugée, le culot resuspendu dans 5 ml acétone, centrifugé, le culot resuspendu dans 5 ml acétone, centrifugé, le culot resuspendu dans 5 ml acétone, centrifugé, le culot laissé à sécher. Le résidu ainsi obtenu a été dissous dans 1 ml d'une solution de NH4HCO3 à 50 mM pour être lyophilisé. Le lyophilisat a été dissous dans 2 ml d'acide chlorhydrique 6N contenant 2 g/l de phénol, le mélange scellé dans une ampoule, puis incubé à 110°C pendant 20 h. La concentration des acides aminés de l'hydrolysat a ensuite été quantifiée par dérivatisation par la ninhydrine suivant les instructions préconisées par le fournisseur de l'analyseur Beckman 6300. L'amino-butyrate a été détecté dans l'hydrolysat des protéines seulement dans le cas où de l'amino-butyrate avait été ajouté au milieu de culture, et seulement pour la souche β5498. La proportion d'amino-butyrate remplaçait un quart de la quantité de valine, correspondant à environ 5 résidus amino-butyrate pour 100 acides aminés des protéines totales. Les résultats détaillés des analyses pour les deux souches CU505 et β5498 dans les deux conditions de culture sont donnés dans le tableau ci-après.

Composition chimique des protéines extraites de souches auxotrophes pour la valine et cultivées en limitation de valine, avec ou sans amino-butyrate

| Acide aminé incorporé dans les protéines | CU505 wt valS -Abu | β5498 valS T222P -Abu | CU505 wtvalS +Abu | β5498 valS T222P + Abu |
|---|---|---|---|---|
| Abu | 0 | 0 | 0 | 0,20 |
| Val | 0,83 | 0,79 | 0,83 | 0,61 |
| Val + Abu | 0,83 | 0,79 | 0,83 | 0,81 |
| Ala | 1,32 | 1,28 | 1,32 | 1,22 |
| Ile | 0,61 | 0,61 | 0,61 | 0,61 |

Résultats exprimés en équivalents Leu

### EXEMPLE 8 : Sélection de nouveaux mutants du code génétique à partir d'une souche mutatrice par isolement sur milieu solide.

La souche β5419, exprimant l'allèle inactif thyA:Val146 à partir d'un plasmide et portant le marqueur ÆnrdD::kan dans le chromosome, ainsi qu'en rend compte sa construction décrite dans l'Exemple 5, a été transduite à l'aide d'un lysat du phage P1 récolté sur la souche TAD, portant un marqueur mutateur ÆmutS::spc, conférant la résistance à la spectinomycine, en sélectionnant sur milieu solide LB contenant de la spectinomycine (25 mg/l) pour obtenir la souche β5555. Le phénotype mutateur de cette souche a été démontré en dénombrant la fréquence des mutants résistant à la rifamycine. En suivant la procédure expérimentale décrite dans l'Exemple 5, des clones capables de croître à 30°C en milieu minéral glucose sans thymidine en présence de 2 à 5 mM de S-carbamoyl-L-cystéine (SCC), ont été obtenus. Trois d'entre ces clones ont servi à préparer des lysats du phage P1, qui ont été utilisés pour transduire la souche β5366, en sélectionnant pour la résistance à la kanamycine, suivant la procédure de l'Exemple 5. Pour chacun des trois lysats, environ la moitié des transductants était capable de croître en milieu solide minéral glucose contenant 3 mM de SCC, indiquant la proximité d'une mutation supprimant l'allèle faux-sens thyA:C146V et du marqueur nrdD::kan. Le locus valS des trois souches β5479, β5485 et β5486, chacune correspondant à un transductant SCC-suppressible obtenu à partir d'un des trois lysats a été amplifié par PCR et séquencé comme il est décrit dans l'Exemple 3. Une mutation ponctuelle différente a été trouvée pour chacune des trois souches, à savoir Arg 223 changée en His dans la souche β5479, Val 276 changée en Ala dans la souche β5485 et Asp 230 changée en Asn dans la souche β5486. Ainsi, chaque clone présentant un phénotype de suppression du mutant faux-sens Cys 146 Val de thyA, présente également la sensibilité à l'amino-butyrate. Chacun de ces clones s'est révélé porter une mutation ponctuelle différente dans le gène valS, validant le crible sélectif comme moyen de diversifier l'activité de la valyl-tRNA synthétase chez Escherichia coli.

Les souches mutantes *E*. *coli* référencée β5456, β5520 et β5498, ont été obtenues à partir de la souche mutante β5419, telle que ci-avant mentionnée à l'Exemple 5, et selon les procédés de sélection tels que décrits ci-avant dans les Exemples 5 et 8.

### BIBLIOGRAPHIE

BAIN J.D., E.S. DIALA, C.G. GLABE, D.A. WACKER, M.H. LYTTLE, T.A. DIX and A.R. CHAMBERLIN, 1991 ; Site-specific incorporation of nonstructural residues during in vitro protein biosynthesis with semisynthetic aminoacyl-tRNAs, Biochemistry 30:5411-5421.
BOUZON, M. and P. MARLIÈRE, 1997 ; Human deoxycytidine kinase as a conditional mutator in Escherichia coli. C.R. Acad.Sci. Paris 320:427-434.
KUNKEL, T.A., and J.D. ROBERTS, 1987 ; Rapid and efficient site-specific mutagenesis without phenotypic selection. Methods Enzymol. 154:367-382.
LEMEIGNAN, B., P. SONIGO and P. MARLIERE, 1993 ; Phenotypic suppression by incorporation of an alien amino acid. J. Mol. Biol. 231:161-166.
LEVH, T.F., J.C.TAYLOR and G.D. MARKHAM, 1988 ; The sulfate activation locus of Escherichia coli K12: cloning, genetic, and enzymatic characterisation. J. Biol. Chem. 263:2409-2416.
PARSOT, C., 1986 ; Evolution of biosynthetic pathways: a common ancestor for threonine synthase, threonine dehydratase and D-serine dehydratase. EMBO J., 5:3013-3019.
RICHAUD, C., D. MENGIN-LECREULX, S. POCHET, E.J. JOHNSON, G.N. COHEN et al., 1993 ; Directed Evolution of Biosynthetic pathways. J. Biol. Chem. 268:26827-26835.
SAMBROOK, J., E.F. FRITSCH and T. MANIATIS, 1989 ; Molecular cloning: A Laboratory Manual. Cold Spring Harbor Laboratory Press, Cold Spring Harbor, NY.
SANGER, F., S. NICKLEN and A.R. COULSON, 1977 ; DNA sequencing with chain-terminating inhibitors. Proc. Natl. Acad. Sci. USA. 74:5463-5467.
SHAPIRO, J.A 1990 ; Action of a transposable element in coding sequence fusions. Genetics 126:293-299.

## Revendications

1. Cellule obtenue par une méthode permettant à des cellules d'acquérir la capacité de produire une protéine dont la séquence d'acides aminés comprend au moins un acide aminé non conventionnel, ladite méthode comprenant les étapes suivantes :
a) la transformation desdites cellules par au moins une introduction d'une mutation faux-sens au niveau d'un codon cible d'un gène codant pour une protéine nécessaire à la croissance desdites cellules, ladite protéine synthétisée à partir du gène ainsi muté n'étant plus fonctionnelle ;
b) le cas échéant la culture des cellules obtenues à l'étape a) dans un milieu de culture contenant un nutriment compensant la perte de fonctionnalité de ladite protéine ainsi mutée ; et
c) la culture des cellules obtenues à l'étape a) ou b) dans un milieu de culture contenant l'acide aminé codé par ledit codon cible,
**caractérisée en ce qu'**elle est choisie parmi les cellules suivantes déposées à la CNCM (Collection Nationale de Culture de Microorganismes, Paris, France)
a) souche *E*. *coli* déposée à la CNCM sous le N° 1-2468 le 28 avril 2000,
b) souche *E*. *coli* déposée à la CNCM sous le N° 1-2469 le 28 avril 2000, et
c) souche *E*. *coli* déposée à la CNCM sous le N° I-2470 le 28 avril 2000.

2. Utilisation d'une cellule selon la revendication 1 pour la production de protéine dont la séquence d'acides aminés comprend au moins un acide aminé non conventionnel.

3. Procédé de production d'une protéine dont la séquence d'acides aminés comprend au moins un acide aminé non conventionnel, **caractérisé en ce qu'**il comprend les étapes suivantes :
a) la culture d'une cellule selon la revendication 1 dans un milieu de culture et des conditions de culture permettant la croissance de ladite cellule ; et
b) l'isolement de ladite protéine comprenant au moins un acide aminé non conventionnel à partir du surnageant de culture et/ou du culot cellulaire obtenu à l'étape a).

4. Procédé selon la revendication 3, **caractérisé en ce que** ledit milieu de culture de l'étape a) permettant la croissance de ladite cellule contient ledit acide aminé non conventionnel ou un de ses précurseurs.

5. Procédé selon la revendication 4, **caractérisé en ce que** ledit acide aminé non conventionnel est synthétisé par ladite cellule.

6. Procédé selon la revendication 5, **caractérisé en ce que** la synthèse dudit acide aminé non conventionnel est augmentée par modification génétique de ladite cellule.

7. Procédé selon l'une des revendications 3 à 6, **caractérisé en ce que** ladite cellule est auxotrophe pour l'acide aminé codé par ledit codon cible.

8. Procédé selon l'une des revendications 3 à 6, **caractérisé en ce que** ladite cellule comprend un gène d'intérêt homologue ou hétérologue dont la séquence codante comporte au moins un codon cible.

9. Procédé selon la revendication 8, **caractérisé en ce** l'étape a) comprend les composés nécessaires à l'induction de la synthèse de la protéine codée par ledit gène d'intérêt.

10. Procédé selon la revendication 8 ou 9, **caractérisé en ce que** l'activité biologique de la protéine codée par ledit gène d'intérêt est au moins partiellement conservée après l'incorporation dudit acide aminé non conventionnel au niveau du codon cible dudit gène d'intérêt.

11. Procédé selon l'une des revendications 3 à 10, **caractérisé en ce que** l'acide aminé non conventionnel est choisi parmi les acides aminés non conventionnels de formule I de configuration L 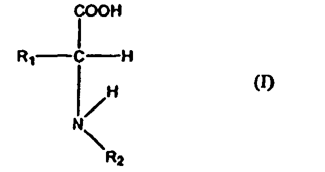 dans laquelle
R₁ ou R₂ représente des radicaux contenant un groupe fonctionnel capable de réagir de manière sélective.

12. Procédé selon la revendication 11, **caractérisé en ce que** le groupe fonctionnel est choisi parmi les groupes aldéhyde, cétone, éthényle, éthynyle ou nitrile.

13. Procédé selon l'une des revendications 4 à 12, pour la fonctionnalisation de protéine.

14. Procédé de purification de protéine, **caractérisé en ce qu'**il comprend les étapes suivantes
a) l'incorporation dans la séquence d'acides aminés de ladite protéine d'un acide aminé non conventionnel contenant un groupe fonctionnel capable de réagir de manière sélective par un procédé selon l'une des revendications 3 à 13 ;
b) la mise en contact de la solution contenant la protéine obtenue à l'étape a) avec un support comprenant un composé capable de réagir spécifiquement avec ledit groupe fonctionnel et de fixer spécifiquement ladite protéine ; et
c) l'isolement de ladite protéine fixée sur le support.

15. Procédé de fixation d'une protéine sur un composé chimique ou biochimique, **caractérisé en ce qu'**il comprend les étapes suivantes
a) l'incorporation dans la séquence d'acides aminés de ladite protéine par un procédé selon l'une des revendications 3 à 13 d'un acide aminé non conventionnel contenant un groupe fonctionnel capable de réagir de manière sélective ;
b) la mise en contact de la protéine obtenue à l'étape a) avec ledit composé chimique ou biochimique comprenant un groupe capable de réagir spécifiquement avec ledit groupe fonctionnel dans un milieu permettant la réaction.

16. Procédé selon la revendication 15, **caractérisé en ce que** ledit composé chimique ou biochimique est lui-même fixé sur un support solide ou est un composé constitutif d'un support solide.

17. Procédé selon la revendication 15 ou 16 pour la préparation d'un complexe protéique.

18. Procédé selon l'une des revendications 15 à 17, **caractérisé en ce que** la protéine fixée ou le composé chimique ou biochimique est choisi parmi les composés thérapeutiques, cosmétiques ou diagnostiques.

19. Procédé selon l'une des revendications 15 à 18, **caractérisé en ce que** le composé chimique ou biochimique est choisi parmi les composés capables de modifier l'activité biologique de la protéine fixée.

20. Procédé selon l'une des revendications 15 à 18, **caractérisé en ce que** le composé chimique ou biochimique est choisi parmi les composés dont l'activité biologique peut être modifiée par la protéine fixée.

21. Procédé selon l'une des revendications 15 à 20, **caractérisé en ce que** le composé chimique ou biochimique est choisi parmi les composés comprenant une protéine, un polynucléotide, un acide gras, un sucre ou un polymère naturel ou synthétique.

22. Protéine obtenue par un procédé selon l'une des revendications 3 à 15, **caractérisée en ce qu'**il s'agit d'une protéine recombinante dont la séquence d'acides aminés comprend au moins un acide aminé non conventionnel choisi parmi les acides aminés non conventionnels de formule I et de configuration L 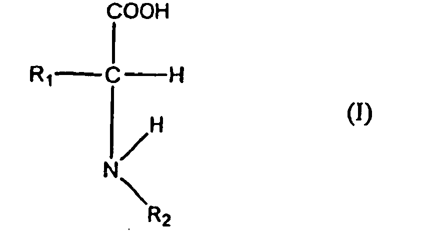 dans laquelle :
R₁ ou R₂ représente des radicaux contenant un groupe fonctionnel capable de réagir de manière sélective.

23. Protéine selon la revendication 22, **caractérisée en ce que** le groupement fonctionnel est choisi parmi les groupes aldéhyde, cétone, éthényle, éthynyle ou nitrile.

24. Complexe protéique obtenu par un procédé selon l'une revendications 15 à 21, **caractérisé en ce qu'**il comprend une protéine recombinante dont la séquence d'acides aminés comprend un acide aminé non conventionnel contenant un groupe fonctionnel, et un composé chimique ou biochimique comprenant un groupe capable de réagir avec ledit groupe fonctionnel.

25. Utilisation d'une protéine selon la revendication 22 ou 23, ou d'un complexe protéique selon la revendication 24 comme réactif de diagnostic.

26. Procédé de diagnostic, **caractérisé en ce qu'**il met en oeuvre une protéine selon la revendication 22 ou 23, ou un complexe protéique selon la revendication 24.

27. Nécessaire de diagnostic, **caractérisé en ce qu'**il contient une protéine selon la revendication 22 ou 23, ou un complexe protéique selon la revendication 24.

28. Utilisation d'une protéine selon la revendication 22 ou 23, d'un complexe protéique selon la revendication 24 ou d'une cellule selon la revendication 1 pour la préparation d'une composition pharmaceutique ou cosmétique.

29. Composition pharmaceutique ou cosmétique comprenant une protéine selon la revendication 22 ou 23, un complexe protéique selon la revendication 24 ou une cellule selon la revendication 1.

## Claims

1. Cell obtained by a method enabling cells to acquire the capacity to produce a protein whereof the amino acid sequence comprises at least one non-conventional amino acid, said method including the following steps:
a) the transformation of said cells by at least one introduction of a false-sense mutation at a target codon of a gene coding for a protein necessary for the growth of said cells, said protein synthesised from the gene thus mutated no longer being functional;
b) where appropriate the culture of the cells obtained at stage a) in a culture medium containing a nutrient compensating for the loss of functionality of said protein thus mutated; and
c) culture of the cells obtained at stage a) or b) in a culture medium containing the amino acid coded by said target codon,
**characterised in that** it is chosen from the following cells deposited in the CNCM (Collection Nationale de Culture de Microoganismes, Paris, France):
a) *E. coli* strain deposited in the CNCM under no. I-2468 on 28 April 2000,
b) *E. coli* strain deposited in the CNCM under no. I-2469 on 28 April 2000, and
c) *E. coli* strain deposited in the CNCM under no. I-2470 on 28 April 2000.

2. Use of a cell according to claim 1, for the production of protein whereof the amino acid sequence comprises at least one non-conventional amino acid.

3. Method for producing a protein whereof the amino acid sequence includes at least one non-conventional amino acid, **characterised in that** it includes the following steps:
a) culture of a cell according to claim 1 in a culture medium and culture conditions allowing the growth of said cell; and
b) isolation of said protein comprising at least one non-conventional amino acid from the culture supernatant and/or the cellular residue obtained at stage a).

4. Method according to claim 3, **characterised in that** said culture medium of stage a) allowing the growth of said cell contains said non-conventional amino acid or one of its precursors.

5. Method according to claim 4, **characterised in that** said non-conventional amino acid is synthesised by said cell.

6. Method according to claim 5, **characterised in that** said non-conventional amino acid is augmented by genetic modification of said cell.

7. Method according to one of claims 3 to 6, **characterised in that** said cell is auxotrophic for the amino acid coded by said target codon.

8. Method according to one of claims 3 to 6, **characterised in that** said cell contains a gene of homologous or heterologous interest, whereof the coding sequence includes at least one target codon.

9. Method according to claim 8, **characterised in that** stage a) includes the compounds necessary for induction of the synthesis of the protein coded by said gene of interest.

10. Method according to claim 8 or 9, **characterised in that** the biological activity of the protein coded by said gene of interest is at least partially retained after incorporation of said non-conventional amino acid at the target codon of said gene of interest.

11. Method according to one of claims 3 to 10, **characterised in that** the non-conventional amino acid is chosen from the non-conventional amino acids of formula I of configuration L 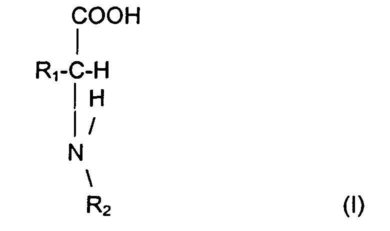 in which:
R₁ or R₂ represents radicals containing a functional group capable of reacting in a selective manner.

12. Method according to claim 11, **characterised in that** the functional group is chosen from the aldehyde, ketone, ethenyl, ethynyl or nitrile groups.

13. Method according to one of claims 4 to 12, for the functionalisation of protein.

14. Method for purifying protein, **characterised in that** it includes the following steps:
a) incorporation in the amino acid sequence of said protein of a non-conventional amino acid containing a functional group capable of reacting in a selective manner by a method according to one of claims 3 to 13;
b) bringing the solution containing the protein obtained at stage a) into contact with a support comprising a compound capable of reacting specifically with said functional group and specifically fixing said protein; and
c) isolation of said protein fixed on the support.

15. Method of fixing a protein on a chemical or biochemical compound, **characterised in that** it comprises the following steps:
a) incorporation in the amino acid sequence of said protein by a method according to one of claims 3 to 13 of a non-conventional amino acid containing a functional group capable of reacting in a selective manner;
b) bringing the protein obtained at stage a) into contact with said chemical or biochemical compound comprising a group capable of reacting specifically with said functional group in a medium allowing the reaction.

16. Method according to claim 15, **characterised in that** said chemical or biochemical compound is itself fixed on a solid support or is a compound constituting a solid support.

17. Method according to claim 15 or 16 for preparing a proteic complex.

18. Method according to one of claims 15 to 17, **characterised in that** the fixed protein or the chemical or biochemical compound is chosen from therapeutic, cosmetic or diagnostic compounds.

19. Method according to one of claims 15 to 18, **characterised in that** the chemical or biochemical compound is chosen from compounds capable of modifying the biological activity of the fixed protein.

20. Method according to one of claims 15 to 1, **characterised in that** the chemical or biochemical compound is chosen from compounds whose biological activity can be modified by the fixed protein.

21. Method according to one of claims 15 to 20, **characterised in that** the chemical or biochemical compound is chosen from the compounds including a protein, a polynucleotide, a fatty acid, a sugar or a natural or synthetic polymer.

22. Protein obtained by a method according to one of claims 3 to 15,
**characterised in that** it concerns a recombinant protein whereof the amino acid sequence includes at least one non-conventional amino acid chosen from the non-conventional amino acids of formula I of configuration L 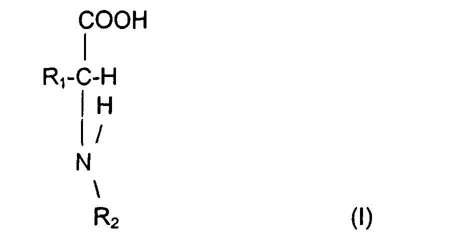 in which:
R₁ or R₂ represents radicals containing a functional group capable of reacting in a selective manner.

23. Protein according to claim 22, **characterized in that** the functional group is chosen from the aldehyde, ketone, ethenyl, ethynyl or nitrile groups.

24. Proteic complex obtained by a method according to one of claims 15 to 21, **characterised in that** it includes a recombinant protein whereof the amino acid sequence includes a non-conventional amino acid containing a functional group and a chemical or biochemical compound comprising a group capable of reacting with said functional group.

25. Use of a protein according to claim 22 or 23, or of a proteic complex according to claim 24 as a diagnostic reagent.

26. Diagnostic method, **characterised in that** it utilises a protein according to claim 22 or 23, or a proteic complex according to claim 24.

27. Diagnostic kit, **characterised in that** it contains a protein according to claim 22 or 23, or a proteic complex according to claim 24.

28. Use of a protein according to claim 22 or 23, of a proteic complex according to claim 24 or a cell according to claim 1 for the preparation of a pharmaceutical or cosmetic composition.

29. Pharmaceutical or cosmetic composition comprising a protein according to claim 22 or 23, a proteic complex according to claim 24 or a cell according to claim 1.

## Patentansprüche

1. Zelle, die durch ein Verfahren erhalten wurde, durch das die Zellen zur Produktion eines Proteins befähigt werden dessen Aminosäuresequenz mindestens eine nicht-konventionelle Aminosäure enthält, wobei das Verfahren folgende Schritte umfasst:
a) Transformation der Zellen durch Einführen mindestens einer Missense-Mutation an einem Zielcodon eines Gens, das für ein für das Zellwachstum notwendiges Protein kodiert, so dass das ausgehend von dem mutierten Gen synthetisierte Protein nunmehr nicht funktionell ist
b) gegebenenfalls Kultivieren der in Schritt a) erhaltenen Zellen in einem Kulturmedium, das ein Nährmittel enthält, welches den Funktionsverlust des auf diese Weise mutierten Proteins kompensiert; und
c) Kultivieren der Zellen, die in Schritt a) oder b) erhalten wurden in einem Kulturmedium, das die Aminosäure enthält, die durch das Zielkodon kodiert wird, **dadurch** charakterisiert, dass sie ausgewählt ist aus folgenden Zellen, welche bei der CNCM (Collection nationale de Culture de Microorganismes, Paris, France) hinterlegt sind
a) Stamm *E. coli* hinterlegt bei CNCM unter der Nummer 1-2468 am 28. April 2000,
b) Stamm *E*. *coli* hinterlegt bei CNCM unter der Nmmer 1-2469 am 28. April 2000, und
c) Stamm *E. coli* hinterlegt bei CNCM unter der Nummer 1-2470 am 28. April 2000.

2. Verwendung einer Zelle gemäß Anspruch 1 für die Herstellung eines Proteins, dessen Aminosäuresequenz mindestens eine nicht-konventionelle Aminosäure enthält.

3. Verfahren zur Herstellung eines Proteins dessen Aminosäuresequenz mindestens eine nicht-konventionelle Aminosäure enthält, **dadurch gekennzeichnet, dass** es folgende Schritte umfasst:
a) Kultivierung einer Zelle gemäß Anspruch 1 in einem Kulturmedium und unter Kulturbedingungen, die das Wachstum der Zelle erlauben; und
b) Isolierung des genannten Proteins, dessen Aminosäuresequenz mindestens eine nicht-konventionelle Aminosäure enthält, aus dem Kulturüberstand und/oder dem Zellanteil, der in Schritt a) erhalten wurde.

4. Das Verfahren gemäß Anspruch 3, **dadurch gekennzeichnet, dass** das Kulturmedium in Schritt a), das das Wachstum der Zelle erlaubt, die nicht-konventionelle Aminosäure oder eines ihrer Vorläufer enthält.

5. Das Verfahren gemäß Anspruch 4, **dadurch gekennzeichnet, dass** die nicht-konventionelle Aminosäure von der genannten Zelle synthetisiert wird.

6. Das Verfahren gemäß Anspruch 5, **dadurch gekennzeichnet, dass** die Synthese der nicht-konventionellen Aminosäure durch genetische Veränderung der genannten Zelle erhöht wird.

7. Das Verfahren gemäß einem der Ansprüche 3 bis 6, **dadurch gekennzeichnet, dass** die Zelle auxotroph bezüglich der durch das Zielkodon kodierten Aminosäure ist.

8. Das Verfahren gemäß einem der Ansprüche 3 bis 6, **dadurch gekennzeichnet, dass** die Zelle ein homologes oder heterologes Gen von Interesse umfasst, dessen Sequenz mindestens ein Zielcodon enthält.

9. Das Verfahren gemäß Anspruch 8, **dadurch gekennzeichnet, dass** Schritt a) alle Substanzen umfasst, die für die Induktion der Synthese des durch das Gen von Interesse kodierten Proteins notwendig sind.

10. Das Verfahren gemäß einem der Ansprüche 8 oder 9, **dadurch gekennzeichnet, dass** die biologische Aktivität des durch das Gen von Interesse kodierten Proteins nach Inkorporation der nicht-konventionellen Aminosäure an der Position des Zielkodons des Gens von Interesse zumindest partiell erhalten bleibt.

11. Das Verfahren gemäß einem der Ansprüche 3 bis 10, **dadurch gekennzeichnet, dass** die nicht-konventionelle Aminosäure ausgewählt ist aus den nicht-konventionellen Aminosäuren gemäß Formel I in Konfiguration L 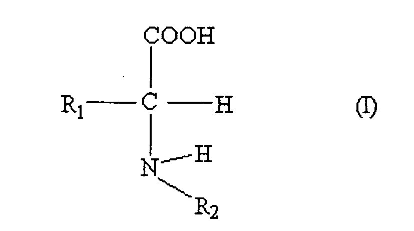 in der R₁ oder R₂ für Radikale stehen, die eine funktionelle Gruppe enthalten, welche selektiv reagieren kann.

12. Das Verfahren gemäß Anspruch 11, **dadurch gekennzeichnet, dass** die funktionelle Gruppe ausgewählt ist aus Aldehydgruppen, Ketogruppen, Ethenylgruppen, Ethynylgruppen oder Nitrilgruppen.

13. Das Verfahren gemäß einem der Ansprüche 4 bis 12, zum Funktionalisieren des Proteins.

14. Verfahren zur Proteinreinigung, **dadurch gekennzeichnet, dass** es folgende Schritte umfasst
a) Inkorporation einer nicht-konventionellen Aminosäure, die eine funktionelle Gruppe enthält, welche selektiv reagieren kann, in die Aminosäuresequenz des genannten Proteins durch ein Verfahren gemäß einem der Ansprüche 3 bis 13;
b) Inkontaktbringen der Lösung, die ein Protein enthält, das in Schritt a) erhalten wurde, mit einem Träger, der eine Verbindung enthält, die imstande ist, mit dieser funktionellen Gruppe selektiv zu reagieren, und das genannte Protein spezifisch zu fixieren; und
c) Isolierung des am Träger fixierten Proteins.

15. Verfahren zur Fixierung eines Proteins an einer chemischen oder biochemischen Verbindung, **dadurch gekennzeichnet, dass** es folgende Schritte umfasst
a) Inkorporation einer nicht-konventionellen Aminosäure, die eine funktionelle Gruppe enthält, welche selektiv reagieren kann, in die Aminosäuresequenz des genannten Proteins durch ein Verfahren gemäß einem der Ansprüche 3 bis 13
b) Inkontaktbringen des Proteins, das in Schritt a) erhalten wurde, mit der chemischen oder biochemischen Verbindung, die eine Gruppe enthält, welche mit der funktionellen Gruppe spezifisch reagieren kann, in einem Milieu, das die Reaktion erlaubt.

16. Das Verfahren gemäß Anspruch 15, **dadurch gekennzeichnet, dass** die chemische oder biochemishce Verbindung selbst auf einem festen Träger fixiert ist oder ein konstitutiver Bestandteil eines festen Trägers ist.

17. Das Verfahren gemäß einem der Ansprüche 15 oder 16, zur Herstellung eines Proteinkomplexes.

18. Das Verfahren gemäß einem der Ansprüche 15 bis 17, **dadurch gekennzeichnet, dass** das fixierte Protein oder die chemische oder biochemische Verbindung ausgewählt ist aus therapeutischen, kosmetischen oder diagnostischen Verbindungen.

19. Das Verfahren gemäß einem der Ansprüche 15 bis 18, **dadurch gekennzeichnet, dass** die chemische oder biochemische Verbindung ausgewählt ist aus Verbindungen, die imstande sind die biologische Aktivität des fixierten Proteins zu modifizieren.

20. Das Verfahren gemäß einem der Ansprüche 15 bis 18, **dadurch gekennzeichnet, dass** die chemische oder biochemische Verbindung ausgewählt ist aus Verbindungen, deren biologische Aktivität vom fixierten Protein modifiziert werden kann.

21. Das Verfahren gemäß einem der Ansprüche 15 bis 20, **dadurch gekennzeichnet, dass** die chemische oder biochemische Verbindung ausgewählt ist aus Verbindungen umfassend ein Protein, ein Polynukleotid, eine Fettsäure, einen Zucker oder ein natürliches oder synthetisches Polymer.

22. Protein, das durch ein Verfahren gemäß einem der Ansprüche 3 bis 15 erhalten wurde, **dadurch gekennzeichnet, dass** es sich um ein rekombinantes Protein handelt, dessen Aminosäuresequenz mindestens eine nicht-konventionelle Aminosäure ausgewählt aus den nicht-konventionellen Aminosäuren gemäß Formel I in Konfiguration L umfasst 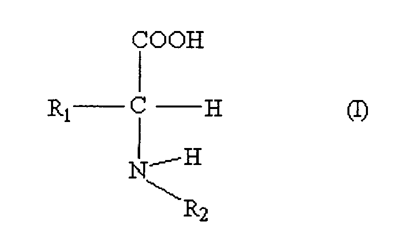 in der R₁ oder R₂ für Radikale stehen, die eine funktionelle Gruppe enthalten, welche selektiv reagieren kann.

23. Das Protein gemäß Anspruch 22, **dadurch gekennzeichnet, dass** die funktionelle Gruppe ausgewählt ist aus Aldehydgruppen, Ketogruppen, Ethenylgruppen, Ethynylgruppen oder Nitrilgruppen.

24. Der Proteinkomplex, der durch ein Verfahren gemäß einem der Ansprüche 15 bis 21 erhalten wurde, **dadurch gekennzeichnet, dass** er ein rekombinantes Protein umfasst, dessen Aminosäuresequenz eine nicht-konventionelle Aminosäure umfasst, die eine funktionelle Gruppe enthält, und eine chemische oder biochemische Verbindung, die eine Gruppe umfasst, welche mit der funktionellen Gruppe reagieren kann.

25. Verwendung eines Proteins gemäß Anspruch 22 oder 23, oder eines Proteinkomplexes gemäß Anspruch 24 als Diagnostikum.

26. Diagnostisches Verfahren, **dadurch gekennzeichnet, dass** ein Protein gemäß einem der Ansprüche 22 oder 23 oder ein Proteinkomplex gemäß Anspruch 24 eingesetzt wird.

27. Diagnostikkit, **dadurch gekennzeichnet, dass** es ein Protein gemäß einem der Ansprüche 22 oder 23, oder einen Proteinkomplex gemäß Anspruch 24 enthält.

28. Verwendung eines Proteins gemäß Anspruch 22 oder 23, eines Proteinkomplexes gemäß Anspruch 24, oder einer Zelle gemäß Anspruch 1 für die Herstellung eines pharmazeutischen Zusammensetzung oder einer kosmetischen Zusammensetzung.

29. Pharmazeutische Zusammensetzung oder kosmetische Zusammensetzung, die ein Protein gemäß einem der Ansprüche 22 oder 23, einen Proteinkomplex gemäß Anspruch 24, oder eine Zelle gemäß Anspruch 1 umfasst.
